Europäisches Patentamt

European Patent Office

Office européen des brevets

(19)

(11) **EP 0 724 481 B1**

(12) **EUROPÄISCHE PATENTSCHRIFT**

(45) Veröffentlichungstag und Bekanntmachung des Hinweises auf die Patenterteilung:
**20.01.1999 Patentblatt 1999/03**

(21) Anmeldenummer: **94929529.9**

(22) Anmeldetag: **11.10.1994**

(51) Int. Cl.$^6$: **B01J 37/08**, C07C 51/25, C07C 57/04

(86) Internationale Anmeldenummer:
**PCT/EP94/03345**

(87) Internationale Veröffentlichungsnummer:
**WO 95/11081 (27.04.1995 Gazette 1995/18)**

(54) **VERFAHREN ZUR HERSTELLUNG VON KATALYTISCH AKTIVEN MULTIMETALLOXIDMASSEN, DIE ALS GRUNDBESTANDTEILE DIE ELEMENTE V UND Mo IN OXIDISCHER FORM ENTHALTEN**

METHOD FOR THE PRODUCTION OF CATALYTICALLY ACTIVE MULTI-METAL OXIDE COMPOSITIONS CONTAINING THE ELEMENTS V AND Mo IN OXIDE FORM AS THEIR MAIN COMPONENTS

PROCEDE DE FABRICATION DE SUBSTANCES FORMEES D'OXYDES MULTIMETALLIQUES A ACTIVITE CATALYTIQUE, RENFERMANT COMME CONSTITUANTS DE BASE LES ELEMENTS V ET Mo SOUS FORME D'OXYDE

(84) Benannte Vertragsstaaten:
**BE DE ES FR GB NL**

(30) Priorität: **21.10.1993 DE 4335973**

(43) Veröffentlichungstag der Anmeldung:
**07.08.1996 Patentblatt 1996/32**

(73) Patentinhaber:
**BASF AKTIENGESELLSCHAFT**
**67056 Ludwigshafen (DE)**

(72) Erfinder:
• **TENTEN, Andreas**
  **D-67434 Neustadt (DE)**
• **HAMMON, Ulrich**
  **D-68165 Mannheim (DE)**
• **WEIDLICH, Peter**
  **D-68165 Mannheim (DE)**

(56) Entgegenhaltungen:
**EP-A- 0 113 156          DE-A- 3 119 586**
**FR-A- 2 248 083**

• **DATABASE WPI Week 7512, Derwent Publications Ltd., London, GB; AN 75-20002 & JP,A,49 097 793 (ASAHI CHEMICAL IND CO) 19. September 1974**
• **DATABASE WPI Week 8322, Derwent Publications Ltd., London, GB; AN 83-52572 & JP,A,58 067 643 (MITSUBISHI RAYON KK) 22. April 1983**

Bemerkungen:
Die Akte enthält technische Angaben, die nach dem Eingang der Anmeldung eingereicht wurden und die nicht in dieser Patentschrift enthalten sind.

**Beschreibung**

Die vorliegende Erfindung betrifft ein Verfahren zur Herstellung von katalytisch aktiven Multimetalloxidmassen, die als Grundbestandteile die Elemente V und Mo in oxidischer Form enthalten, bei dem man aus Ausgangsverbindungen, die die elementaren Konstituenten der Multimetalloxidmassen als Bestandteil enthalten, ein inniges Trockengemisch herstellt und dieses bei einer Temperatur von 300 bis 450°C in einer aus Sauerstoff, Ammoniak sowie als Restmenge Wasserdampf und/oder Inertgas bestehenden Gasatmosphäre calciniert.

Aus Zesz. Nauk.-Politech. Lodz., Chem. 616 (43), 95-106 (1991), ist bekannt, daß sich Mulimetalloxidmassen, die als Grundbestandteile die Elemente V und Mo in oxidischer Form enthalten, als Katalysatoren zur katalytischen Gasphasenoxidation von Acrolein zu Acrylsäure eignen. Ferner ist aus der vorgenannten Schrift bekannt, derartige katalytisch aktive Multimetalloxidmassen so zu erzeugen, daß man Katalysatorvorläufer zunächst in Gegenwart von Luft und anschließend in Gegenwart eines aus Acrolein, Sauerstoff und Inertgasen bestehenden Gasgemisches calciniert. Eine entsprechende Herstellungsempfehlung wird in Catal. Rev.-Sci. Eng., 35(2), 213-259 (1993) gegeben. Nachteilig an dieser Verfahrensweise ist jedoch , daß Acrolein umfassende Calcinationsatmosphären in herkömmlichen Calcinationsöfen nicht handhabbar sind.

Aus der DE-31 19 586 C2 ist bekannt, eine katalytisch aktive Multimetalloxidmasse, die als Grundbestandteile die Elemente V und Mo in oxidischer Form enthält, dadurch herzustellen, daß man aus Ausgangsverbindungen, die die elementaren Konstituenten der Multimetalloxidmassen als Bestandteil enthalten, ein inniges, Ammoniumionen umfassendes, Trockengemisch herstellt, und dieses bei 380°C in einem Gasstrom, der neben Wasserdampf und Inertgasen 1 Vol.-% Sauerstoff enthält, calciniert. Die resultierende Multimetalloxidmasse wird in der DE-31 19 586 C2 als Katalysator zur katalytischen Gasphasenoxidation von Acrolein zu Acrylsäure empfohlen.

Aufgrund des Gehaltes an Ammoniumionen der zu calcinierenden Masse enthält die Calcinationsatmosphäre gemäß der DE-31 19 586 C2 selbstredend Ammoniak. Einen Einfluß des Ammoniak-Gehalts der Calcinationsatmosphäre auf die Katalysatoraktivität und -selektivität sieht die DE-31 19 586 C2 nicht.

DATABASE WPI, Week 7512, Derwent Publication Ltd., London, GB; AN 75-20002 & JP-A-49 097 793 (Asahi Chemical Ind. Co.) 19. September 1974 empfiehlt die Calcination von relevanten Multimetalloxidkatalysatoren unter völligem $O_2$-Ausschluß, aber im Beisein von $NH_3$.

Die EP-A 113 156 empfiehlt die Calcination von relevanten Multimetalloxidmassen im Beisein einer auf 0,05 bis 3 Vol.-% begrenzten Ammoniakmenge sowie im Luftstrom.

Der vorliegenden Erfindung liegt nun die überraschende Entdeckung zugrunde, daß bei V und Mo als Grundbestandteilen in oxidischer Form enthaltenden Multimetalloxidmassen ein definierter $NH_3$-Gehalt der Calcicationsatmosphäre bei gleichzeitig definiertem $O_2$-Gehalt derselben zu katalytisch aktiven Massen führt, die bei Verwendung als Katalysatoren zur gasphasenkatalytisch oxidativen Gewinnung von Acrylsäure aus Acrolein eine erhöhte Katalysatoraktivität und -selektivität aufweisen.

Demgemäß wurde ein Verfahren zur Herstellung von katalytisch aktiven Multimetalloxidmassen, die als Grundbestandteile die Elemente V und Mo in oxidischer Form enthalten, gefunden, bei dem man aus Ausgangsverbindungen, die die elementaren Konstituenten der Multimetalloxidmassen als Bestandteil enthalten, ein inniges Trockengemisch herstellt und dieses bei einer Temperatur von 300 bis 450°C in einer $O_2$ und $NH_3$ aufweisenden Gasatmosphäre calciniert, dadurch gekennzeichnet, daß die Gasatmosphäre, in der die Calcinierung erfolgt, zusammengesetzt ist aus

- zu jedem Zeitpunkt der Calcinierung 0,5 bis 4, vorzugsweise 1 bis 2, Vol.-% $O_2$,

- über die Gesamtdauer der Calcinierung gemittelt 1 bis 8 Vol.-% $NH_3$ sowie

- Wasserdampf und/oder Inertgas als Restmenge.

Vorzugsweise beträgt der über die Gesamtdauer der Calcinierung gemittelte $NH_3$-Gehalt der Calcinationsatmosphäre 1 bis 6 und besonders bevorzugt 4 bis 6 Vol.-%.

Besonders bevorzugt wird die Calcinierung so durchgeführt, daß sie zu 50 bis 95 % der Calcinierungsgesamtdauer (in der Regel 3 bis 15 h) bei einer Calcinierungstemperatur von 300 bis 350°C (Calcinierungsstufe I) und zu 5 bis 50 % der Calcinierungsgesamtdauer in einer darauffolgenden Calcinierungsstufe II bei einer Calcinierungstemperatur von 380 bis 450°C erfolgt.

Dabei ist es von Vorteil, wenn der über die Gesamtdauer der Calcinierungsstufe I gemittelte $NH_3$-Gehalt der Calcinationsatmosphäre 5 bis 8 Vol.-% und der über die Gesamtdauer der Calcinierungsstufe II gemittelte $NH_3$-Gehalt der Calcinationsatmosphäre $\leq 4$ Vol.-%, vorzugsweise 1 bis 3 Vol.-%, beträgt.

Mit besonderem Vorteil werden die vorgenannten numerischen $NH_3$-Gehalte nicht nur als zeitliche Mittelwerte erfüllt, sondern liegen als solche zu jedem Zeitpunkt in den Calcinationsatmosphären der Calcinationsstufen I, II vor.

Als günstig erweist sich das erfindungsgemäße Verfahren insbesondere für katalytisch aktive Multimetalloxidmassen mit einem molaren Verhältnis von Mo : V von 12 : 1 bis 6.

Zusätzlich enthalten die erfindungsgemäßen Verfahrensprodukte mit Vorteil W u. /o. Nb, und zwar in einem molaren Verhältnis zu Mo von 0,2 bis 4 : 12. Ferner ist es von Vorteil, wenn die katalytisch aktiven Multimetalloxidmassen über die bereits genannten metallischen Elemente hinaus zusätzlich Cu enthalten, und zwar in einem molaren Verhältnis zu Mo von 0,5 bis 18 : 12.

Besonders günstige erfindungsgemäße Verfahrensprodukte genügen der nachfolgenden allgemeinen Stöchiometrie I

$$Mo_{12} V_a X^1_b X^2_c X^3_d X^4_e X^5_f X^6_g O_n \qquad (I),$$

in der die Variablen folgende Bedeutung haben:

$X^1$    W, Nb, Ta, Cr und/oder Ce,

$X^2$    Cu, Ni, Co, Fe, Mn und/oder Zn,

$X^3$    Sb und/oder Bi,

$X^4$    wenigstens eines oder mehrere Alkalimetalle,

$X^5$    wenigstens eines oder mehrere Erdalkalimetalle,

$X^6$    Si, Al, Ti und/oder Zr,

a    1 bis 6,

b    0,2 bis 4,

c    0,5 bis 18,

d    0 bis 40,

e    0 bis 2,

f    0 bis 4,

g    0 bis 40 und

n    eine Zahl, die durch die Wertigkeit und Häufigkeit der von Sauerstoff verschiedenen Elemente in I bestimmt wird.

Unter diesen sind jene mit nachfolgender Bedeutung für die Variablen bevorzugt:

$X^1$    W, Nb und/oder Cr,

$X^2$    Cu, Ni, Co und/oder Fe,

$X^3$    Sb,

$X^4$    Na und/oder K,

$X^5$    Ca, Sr und/oder Ba,

$X^6$    Si, Al und/oder Ti,

a    2,5 bis 5,

b    0,5 bis 2,

c       0,5 bis 3,

d       0 bis 2,

e       0 bis 0,2,

f       0 bis 1,

g       0 bis 15 und

n       eine Zahl, die durch die Wertigkeit und Häufigkeit der von Sauerstoff verschiedenen Elemente in I bestimmt wird.

Ganz besonders bevorzugt sind jedoch die nachfolgenden Multimetalloxidmassen II als unmittelbare Verfahrensprodukte

$$Mo_{12} V_a X_b^1 X_c^2 X_f^5 X_g^6 O_n \qquad (II),$$

mit

$X^1$      W und/oder Nb,

$X^2$      Cu und/oder Ni,

$X^5$      Ca und/oder Sr,

$X^6$      Si und/oder Al,

a       3 bis 4,5,

b       1 bis 1,5,

c       0,75 bis 2,5,

f       0 bis 0,5,

g       0 bis 8 und

n       eine Zahl, die durch die Wertigkeit und Häufigkeit der von Sauerstoff verschiedenen Elemente in I bestimmt wird.

Im Rahmen der Ausübung des erfindungsgemäßen Verfahrens geht man von in an sich bekannter Weise geeigneten Quellen der Multimetalloxidmassen aus und erzeugt aus diesen ein möglichst inniges, vorzugsweise feinteiliges Trockengemisch, welches dann der Calcinierung unterworfen wird, wobei die Calcinierung vor oder nach der Formung zu Katalysatorkörpern bestimmter Geometrie erfolgen kann. Wesentlich ist, wie allgemein bekannt, nur, daß es sich bei den Quellen entweder bereits um Oxide handelt, oder um solche Verbindungen, die durch Erhitzen, wenigstens in Anwesenheit von Sauerstoff, in Oxide überführbar sind. Neben den Oxiden kommen daher als Ausgangsverbindungen vor allem Halogenide, Nitrate, Formiate, Oxalate, Acetate, Carbonate oder Hydroxide in Betracht. Geeignete Ausgangsverbindungen des Mo, V, W und Nb sind auch deren Oxoverbindungen (Molybdate, Vanadate, Wolframate und Niobate) bzw. die von diesen abgeleiteten Säuren.

Das innige Vermischen der Ausgangsverbindungen kann in trockener oder in nasser Form erfolgen. Erfolgt es in trockener Form, werden die Ausgangsverbindungen zweckmäßigerweise als feinteilige Pulver eingesetzt und nach dem Mischen z.B. zu Katalysatorkörpern gewünschter Geometrie verpreßt (z.B. tablettiert), die dann der Calcinierung unterworfen werden.

Vorzugsweise erfolgt das innige Vermischen jedoch in nasser Form. Üblicherweise werden die Ausgangsverbindungen dabei in Form einer wäßrigen Lösung oder Suspension miteinander vermischt. Anschließend wird die wäßrige Masse getrocknet und nach Trocknung calciniert. Vorzugsweise erfolgt der Trocknungsprozeß unmittelbar im Anschluß an die Fertigstellung der wäßrigen Mischung und durch Sprühtrocknung (die Austrittstemperaturen betragen in der

4

Regel 100 bis 150°C). Das dabei anfallende Pulver kann unmittelbar durch Pressen geformt werden. Häufig erweist es sich jedoch für eine unmittelbare Weiterverarbeitung als zu feinteilig, weshalb es dann unter Zusatz von Wasser zweckmäßigerweise geknetet wird.

Die anfallende Knetmasse wird anschließend entweder zur gewünschten Katalysatorgeometrie geformt, getrocknet und dann der Calcinierung unterworfen (führt zu sogenannten Vollkatalysatoren) oder ungeformt calciniert und danach zu einem feinteiligen (üblicherweise < 80 μm) Pulver vermahlen, welches normalerweise unter Zusatz einer geringen Menge Wasser sowie gegebenenfalls weiterer üblicher Bindemittel als Feuchtmasse auf inerte Trägerkörper aufgetragen wird. Nach beendeter Beschichtung wird nochmals getrocknet und so der einsatzfähige Schalenkatalysator erhalten. Prinzipiell kann das calcinierte Pulver aber auch als Pulverkatalysator eingesetzt werden. Erfolgt das Vermischen der Ausgangsverbindungen in Form einer wäßrigen Lösung, so können mit selbiger auch inerte poröse Trägerkörper getränkt, getrocknet und anschließend zu Trägerkatalysatoren calciniert werden.

Bei der Herstellung von Schalenkatalysatoren kann das Beschichten der Trägerkörper auch vor der Calcinierung, d.h. z.B. mit dem befeuchteten Sprühpulver, erfolgen. Für Schalenkatalysatoren geeignete Trägermaterialien sind z.B. poröse oder unporöse Aluminiumoxide, Siliciumdioxid, Thoriumdioxid, Zirkondioxid, Siliciumcarbid oder Silikate wie Magnesium- oder Aluminiumsilicat. Die Trägerkörper können regelmäßig oder unregelmäßig geformt sein, wobei regelmäßig geformte Trägerkörper mit deutlich ausgebildeter Oberflächenrauhigkeit, z.B. Kugeln oder Hohlzylinder, bevorzugt werden. Unter diesen sind wiederum Kugeln von Vorteil.

Ganz besonders vorteilhaft ist die Verwendung von im wesentlichen unporösen, oberflächenrauhen, kugelförmigen Trägern aus Steatit, deren Durchmesser 1 bis 6 mm, bevorzugt 4 bis 5 mm beträgt. Die Schichtdicke der Aktivmasse wird zweckmäßigerweise als im Bereich 50 bis 500 μm, bevorzugt im Bereich 100 bis 250 μm liegend gewählt.

Die erfindungsgemäß erforderliche Calcinationsatmosphäre läßt sich in einfacher Weise beispielsweise dadurch realisieren, daß man in einem Ofen calciniert durch den man ein Gasgemisch führt, das die entsprechende Zusammensetzung bezüglich $O_2$, $NH_3$ und Inertgasen/Wasserdampf aufweist. In einer weniger bevorzugten Ausführungsform kann der erforderliche mittlere Ammoniakgehalt der Calcinierungsatmosphäre auch dadurch realisiert werden, daß man in die zu calcinierende Trockenmasse eine entsprechende Menge Ammoniumionen einarbeitet, welche sich im Verlauf der Calcinierung unter $NH_3$-Abgabe zersetzen. Eine darüber hinausgehende Zufuhr an $NH_3$ ist erfindungsgemäß nicht unabdingbar. Zweckmäßigerweise lassen sich die Ammoniumionen in die Trockenmischung dadurch einbringen, daß man als Quellen der Elemente Mo, V, W oder Nb die entsprechenden Ammoniumoxometallate verwendet. Beispiel hierfür sind Ammoniummetaniobat, Ammoniummetavanadat, Ammoniumheptamolybdattetrahydrat und Ammoniumparawolframatheptahydrat. Selbstverständlich können in die zu calcinierende Trockenmischung aber auch unabhängig von den als Quellen der Katalysatorkonstituenten erforderlichen Ausgangsverbindungen Ammoniumlieferanten wie $NH_4NO_3$ oder Ammoniumacetat eingearbeitet werden, die beim Calcinieren in vollem Umfang zu gasförmigen Verbindungen zerfallen.

Die lediglich Einarbeitung von Ammoniumionen in das zu calcinierende Trockengemisch ist erfindungsgemäß jedoch nicht ausreichend. Vielmehr stellt sich der erforderliche mittlere Ammoniakgehalt der Calcinationsatmosphäre nur dann ein, wenn die zu calcinierende Katalysatormenge darüber hinaus in entsprechender Weise an das Innenvolumen des Calcinierungsofens angepaßt wird. Selbstverständlich lassen sich diese $NH_3$-Entwicklung aus dem Katalysatorvorläufer heraus und eine externe $NH_3$-Zufuhr auch kombiniert anwenden. Als Calcinierungsofen wird man zweckmäßigerweise einen mit den entsprechenden Gasgemischen beschickten Umluftofen einsetzen. Bei alleiniger $NH_3$-Entwicklung aus dem Katalysatorvorläufer (der zu calcinierenden Trockenmischung) heraus, durchläuft der $NH_3$-Gehalt der Calcinierungsatmosphäre als Funktion der Calcinationsdauer (in der Regel wenige Stunden; mit der Calcinationstemperatur fällt die erforderliche Calcinationsdauer) normalerweise ein Maximum. Befriedigende Katalysatoraktivitäten werden dabei insbesondere dann erhalten, wenn der $NH_3$-Gehalt der Calcinationsatmosphäre als Funktion der Calcinationsdauer wie folgt verläuft:

| Calcinationsdauer (ausgedrückt in % der Gesamtdauer) | Vol.-% $NH_3$ |
|---|---|
| 0 - 10 | 0 - 5 |
| >10 - 15 | 3 - 10 |
| >15 - 25 | 8 - 16 |
| >25 - 40 | 6 - 14 |
| >40 - 60 | 1,5 - 8 |
| >60 - 100 | 0 - 2 |

Besonders wohldefinierte Calcinationsbedingungen lassen sich bei Verwendung eines Bandcalcinationsofens realisieren. Unter dem Begriff "Intergase" werden all jene Gase verstanden, die mit der zu calcinierenden Masse während der Calcination keine chemische Reaktion eingehen. Beispiele für Inertgase sind $N_2$ oder Edelgase. Wasserdampf wird die Calcinationsatmosphäre insbesondere dann aufweisen, wenn der Katalysatorvorläufer Hydratwasser enthält.

In der Regel wird der Wasserdampfanteil der Calcinationsatmosphäre zu keinem Zeitpunkt während der Calcinierung 20 Vol.-% überschreiten.

Die nach dem erfindungsgemäßen Verfahren resultierenden Multimetalloxidmassen eignen sich insbesondere als Katalysatoren zur katalytischen Gasphasenoxidation von Acrolein zu Acrylsäure. Sie weisen bezüglich dieser Umsetzung eine erhöhte Aktivität auf und bewirken eine Acrylsäurebildung in erhöhter Selektivität. Dies wird darauf zurückgeführt, daß die spezifischen Calcinationsbedingungen insbesondere bezüglich der Elemente V und Mo zu einer besonders vorteilhaften Verteilung verschiedener Oxidationsstufen in den erfindungsgemäß resultierenden Multimetalloxidmassen führen. Die katalytische Gasphasenoxidation von Acrolein zu Acrylsäure erfolgt dabei in an sich bekannter Weise. Der im Rahmen der Gasphasenoxidation benötigte Sauerstoff kann z.B. in Form von Luft, aber auch in reiner Form zugesetzt werden. Aufgrund der hohen Reaktionswärme werden die Reaktionspartner vorzugsweise mit Inertgas wie $N_2$, $CO_2$, niederen Kohlenwasserstoffen, rückgeführten Reaktionsabgasen und/oder Wasserdampf verdünnt. Üblicherweise wird bei der Acroleinoxidation ein Acrolein : Sauerstoff : Wasserdampf : Inertgas-Volumenverhältnis von 1 : (1 bis 3) : (0 bis 20) : (3 bis 30), vorzugsweise von 1 : (1 bis 3) : (0,5 bis 10) : (7 bis 18) eingestellt. Normalerweise wird bei dem Verfahren Acrolein eingesetzt, das durch die katalytische Gasphasenoxidation von Propen erzeugt wurde. In der Regel werden die Acrolein enthaltenden Reaktionsgase dieser Propenoxidation ohne Zwischenreinigung eingesetzt. Der Reaktionsdruck beträgt in der Regel 1 bis 3 bar und die Gesamtraumbelastung beträgt vorzugsweise 1.000 bis 3.500 Nl/l/h. Typische Vielrohr-Festbettreaktoren sind z.B. in den Schriften DE-A 28 30 765, DE-A 22 01 528 oder US-A 3 147 084 beschrieben.

Neben der gasphasenkatalytischen Oxidation von Acrolein zu Acrylsäure vermögen die erfindungsgemäßen Verfahrensprodukte aber auch die gasphasenkatalytische Oxidation anderer organischer Verbindungen wie insbesondere anderer, vorzugsweise 3 bis 6 C-Atome aufweisender, Alkane, Alkanole, Alkanale, Alkene und Alkenole (z.B. Propylen, Methacrolein, tert.-Butanol, Methylether des tert.-Butanol, iso-Buten, iso-Butan oder iso-Butyraldehyd) zu olefinisch ungesättigten Aldehyden und/oder Carbonsäuren, sowie den entsprechenden Nitrilen (Ammoxidation, vor allem von Propen zu Acrylnitril und von iso-Buten bzw. tert.-Butanol zu Methacrylnitril) zu katalysieren. Beispielhaft genannt sei die Herstellung von Acrolein, Methacrolein und Methacrylsäure. Sie eignen sich aber auch zur oxidativen Dehydrierung olefinischer Verbindungen.

Umsatz, Selektivität und Verweilzeit sind an dieser Schrift, falls nichts anderes erwähnt wird, wie folgt definiert:

6

EP 0 724 481 B1

Umsatz U an Acrolein (%) =

$$\frac{\text{Molzahl umgesetztes Acrolein}}{\text{Molzahl eingesetztes Acrolein}} \times 100 \text{ ;}$$

Selektivität der Acrylsäurebildung (%) =

$$\frac{\text{Molzahl Acrolein umgesetzt zu Acrylsäure}}{\text{Molzahl Acrolein insgesamt umgesetzt}} \times 100 \text{ ;}$$

Verweilzeit (sec) =

$$\frac{\text{mit Katalysator gefülltes Leervolumen des Reaktors (l)}}{\text{durchgesetzte Synthesegasmenge (Nl/h)}} \times 3600 \text{ ;}$$

Eine unter ansonsten unveränderten Reaktionsbedingungen bei tieferer Temperatur zum gleichen Umsatz führende Aktivmasse verfügt über eine erhöhte Aktivität.

Beispiele

a) Herstellung eines Katalysatorvorläufers
190 g Kupfer(II)acetatmonohydrat wurden in 2700 g Wasser zu einer Lösung I gelöst. In 5.500 g Wasser wurden bei 95°C nacheinander 860 g Ammoniumheptamolybdattetrahydrat, 143 g Ammoniummetavanadat und 126 g Ammoniumparawolframatheptahydrat zu einer Lösung II gelöst. Anschließend wurde die Lösung I auf einmal in die Lösung II eingerührt und das wäßrige Gemisch bei einer Austrittstemperatur von 110°C sprühgetrocknet. Danach wurde das Sprühpulver je kg Pulver mit 0,15 kg Wasser verknetet.

b) Calcinierung
Der unter a) erhaltene Katalysatorvorläufer wurde in einem mit einem Sauerstoff/Stickstoff-Gemisch beschickten Umluftofen calciniert. Der Sauerstoffgehalt wurde dabei in allen Fällen so eingestellt, daß am Ausgang des Umluftofens ein $O_2$-Gehalt von 1,5 Vol.-% bestand. Im Rahmen der Calcinierung wurde die Knetmasse aus a) zunächst mit einer Geschwindigkeit von 10 K/min auf 300°C aufgeheizt und anschließend während 6 h auf dieser Temperatur gehalten. Danach wurde mit einer Geschwindigkeit von 10 K/min auf 400°C aufgeheizt und diese Temperatur noch 1 h aufrechterhalten. Zur Realisierung unterschiedlicher Ammoniakgehalte der Calcinierungsatmopshäre wurde einerseits die Ofenbeladung O (g Katalysatorvorläufer pro l Innenvolumen des Umluftofens), der Eingangsvolumenstrom ES (Nl/h) des Sauerstoff/Stickstoff-Gemisches und die Verweilzeit VZ (sec) der Sauerstoff/Stickstoff-Beschickung (Verhältnis aus Innenvolumen des Umluftofens und Volumenstrom des zugeführten Sauerstoff/Stickstoff-Gemisches) variiert. Ferner enthielt das zugeführte Sauerstoff/Stickstoff-Gasgemisch in einigen Fällen einen bestimmten Volumenanteil VA an $NH_3$ (Vol.-%) . Die verwendeten Umluftöfen wiesen Innenvolumina von 3 l bzw. 40 l auf.

c) Katalytische Gasphasenoxidation von Acrolein zu Acrylsäure
Das in b) calcinierte, katalytisch aktive Material wurde auf Teilchendurchmesser von 0,1 bis 50 µm gemahlen. Mit dem so erhaltenen Aktivmassenpulver wurden in einer Drehtrommel unporöse oberflächenrauhe Steatitkugeln eines Durchmessers von 4 bis 5 mm in einer Menge von 50 g Pulver je 200 g Steatitkugeln bei gleichzeitigem Zusatz von 18 g Wasser beschichtet. Anschließend wurde mit 110°C heißer Luft getrocknet. Der so erhaltene Schalenkatalysator wurde mit einer konstanten Menge Inertmaterial verdünnt unter folgenden Reaktionsbedingungen in einem Rohrbündelreaktor zur katalytischen Gasphasenoxidation von Acrolein zu Acrylsäure eingesetzt:

Zusammensetzung des Reaktionsgases:

5 Vol.-% Acrolein, 7 Vol.-% $O_2$, 10 Vol.-% $H_2O$ und $N_2$ als Restmenge;
Raumbelastung: 2.300 Nl/l/h.

Die Temperatur des zur Temperierung verwendeten Salzbades wurde so gewählt, daß bei einfachem Durchgang eine Acroleinumwandlung von ca. 99 mol.-% resultierte.

Die nachfolgende Tabelle zeigt die für unter verschiedenen Calcinationsbedingungen erhaltenen katalytisch aktiven Massen erforderlichen Salzbadtemperaturen (BTemp.) sowie die Selektivität der Acrylsäurebildung (S [%]), nach 4 Wochen Betriebsdauer. Je höher die erforderliche Salzbadtemperatur ist, desto niedriger ist die Katalysatoraktivität. Ferner reflektiert die Tabelle die Calcinierungsbedingungen, insbesondere den mittleren $NH_3$-Gehalt der Calcinierungsatmosphäre ($\overline{NH_3}$ [Vol.-%]).

Tabelle

| Beispiele B, Vergleichs-bsp. V | I (l) | O (g/l) | VZ (sec) | ES (Nl/h) | VA (Vol.-%) | B Temp. (°C) | S (%) | $\overline{NH_3}$ (Vol.-%) |
|---|---|---|---|---|---|---|---|---|
| B1 | 3 | 250 | 86 | 125 | – | 260 | 94,8 | 4 |
| B2 | 3 | 50 | 86 | 125 | – | 272 | 93,9 | 1 |
| B3 | 3 | 250 | 43 | 250 | – | 265 | 94,2 | 2 |
| B4 | 3 | 250 | 135 | 80 | – | 257 | 95,3 | 6 |
| V1 | 40 | 19 | 78 | 1850 | – | 275 | 93,5 | 0,5 |
| B5 | 3 | 250 | 86 | 125 | 2 | 255 | 95,3 | 6 |
| V2 | 3 | 250 | 86 | 125 | 8 | 279 | 92,7 | 12 |

**Patentansprüche**

1. Verfahren zur Herstellung von katalytisch aktiven Multimetalloxidmassen, die als Grundbestandteile die Elemente V und Mo in oxidischer Form enthalten, bei dem man aus Ausgangsverbindungen, die die elementaren Konstituenten der Multimetalloxidmassen als Bestandteil enthalten, ein inniges Trockengemisch herstellt und dieses bei einer Temperatur von 300 bis 450°C in einer $O_2$ und $NH_3$ aufweisenden Gasatmosphäre calciniert, dadurch gekennzeichnet, daß die Gasatmosphäre, in der die Calcinierung erfolgt, zusammengesetzt ist aus

   - zu jedem Zeitpunkt der Calcinierung 0,5 bis 4 Vol.-% $O_2$,

   - über die Gesamtdauer der Calcinierung gemittelt 1 bis 8 Vol.-% $NH_3$ sowie

   - Wasserdampf und/oder Inertgas als Restmenge.

2. Verfahren nach Anspruch 1, dadurch gekennzeichnet, daß die Calcinierung so durchgeführt wird, daß sie zu 50 bis 95 % der Calcinierungsgesamtdauer bei einer Calcinierungstemperatur von 300 bis 350°C (Calcinierungsstufe I) und zu 5 bis 50 % der Calcinierungsgesamtdauer in einer darauffolgenden Calcinierungsstufe II bei einer Calcinierungstemperatur von 380 bis 450°C erfolgt, wobei der über die Gesamtdauer der Calcinierungsstufe I gemittelte $NH_3$-Gehalt der Calcinationsatmosphäre 5 bis 8 Vol.-% und der über die Gesamtdauer der Calcinierungsstufe II gemittelte $NH_3$-Gehalt der Calcinationsatmosphäre $\leq$ 4 Vol.-% beträgt.

**Claims**

1. A process for the preparation of a catalytically active poly-metal oxide material which contains the elements V and Mo in oxide form as basic components, in which an intimate dry mixture is prepared from starting compounds which contain the elemental constituents of the poly-metal oxide material as a component and said mixture is calcined at from 300 to 450°C in a gas atmosphere comprising $O_2$ and $NH_3$, wherein the gas atmosphere in which the calcination is carried out is composed of

   - from 0.5 to 4% by volume of $O_2$ throughout the calcination,

   - from 1 to 8% by volume, averaged over the total duration of the calcination, of $NH_3$ and

   - steam or inert gas as the remaining amount.

2. A process as claimed in claim 1, wherein the calcination is carried out at a calcination temperature of from 300 to 350°C for from 50 to 95% of the total calcination time (calcination stage I) and at a calcination temperature of from 380 to 450°C for from 5 to 50% of the total calcination time in a subsequent calcination stage II, the $NH_3$ content of the calcination atmosphere averaged over the total duration of calcination stage I being from 5 to 8% by volume and the $NH_3$ content of the calcination atmosphere averaged over the total duration of calcination stage II being $\leq$ 4% by volume.

**Revendications**

1. Procédé de préparation de masses d'oxydes multimétalliques à action catalytique contenant en tant qu'ingrédients de base les éléments V et Mo sous forme oxydée, dans lequel on prépare, à partir de composés de départs contenant comme ingrédient les constituants élémentaires des masses d'oxydes métalliques, un mélange intime à sec qu'on calcine ensuite à une température de 300-450°C dans une atmosphère gazeuse contenant $O_2$ et $NH_3$, caractérisé en ce que l'atmosphère gazeuse dans laquelle la calcination a lieu se compose

   - de 0,5-4% en volume de $O_2$, à chaque instant de la calcination,
   - de 1-8% en volume de $NH_3$ estimé en moyenne sur toute la durée de la calcination,
   - de vapeur d'eau et/ou de gaz inerte pour le complément.

2. Procédé selon la revendication 1, caractérisé en ce que la calcination est effectuée de sorte que 50-95% de la durée totale de la calcination se passe à une température de calcination de 300-350°C (étape I de calcination) et 5-50% de la durée totale de la calcination se passe à une température de calcination de 380-450°C au cours au cours d'une étape II de calcination subséquente, la teneur en $NH_3$ de l'atmosphère de calcination, estimée sur toute la durée de l'étape I de la calcination, s'élevant à 5-8% en volume, et la teneur en NH$_3$ de l'atmosphère de calcination, estimée sur toute la durée de l'étape II de la calcination, étant ≤ 4% en volume.